# EUROPEAN PATENT SPECIFICATION

(11) **EP 2 702 137 B1**
(45) Date of publication and mention of the grant of the patent: **07.12.2016**
(21) Application number: 12777735.7
(22) Date of filing: 25.04.2012
(51) Int. Cl.: C12N 1/14

(54) **METHOD FOR MAKING DEHYDRATED MYCELIUM ELEMENTS AND PRODUCT MADE THEREBY**
VERFAHREN ZUR HERSTELLUNG DEHYDRIERTER MYZELELEMENTE UND DARAUS HERGESTELLTES PRODUKT
PROCÉDÉ DE FABRICATION D'ÉLÉMENTS DE MYCÉLIUM DÉSHYDRATÉS ET PRODUIT FABRIQUÉ PAR CE PROCÉDÉ

(30) Priority: 25.04.2011 US 201161517749 P; 24.04.2012 US 201213454856
(43) Date of publication of application: 05.03.2014
(73) Proprietor: Ecovative Design LLC, Green Island, NY 12183 (US)
(72) Inventor: BAYER, Eben, Troy, NY 12180 (US); MCINTYRE, Gavin, Troy, NY 12180 (US)
(74) Representative: Johnston, Magnus George
(86) International application number: PCT/US2012/034960
(87) International publication number: WO 2012/148995

(56) References cited:
- EP-A2- 0 382 259
- US-A1- 2008 145 577
- US-A1- 2009 307 969
- US-A1- 2011 269 209
- US-A1- 2012 135 504
- US-A1- 2012 227 899
- BOOTH ET AL. POTENTIAL OF A DRIED MYCELIUM FORMULATION OF AN INDIGENOUS STRAIN OF METARHIZIUM ANISOPLIAE AGAINST SUBTERRANEAN PESTS OF CRANBERRY BIOCONTROL SCIENCE AND TECHNOLOGY vol. 10, 2000, pages 659 - 668, XP008170983

## Description

This invention relates to a method for making dehydrated mycelium elements.

As is known from published United States Patent Application 2008/0145577, use can be made of a fungus to form composite materials by mixing an inoculum including a preselected fungus with discrete particles and a nutrient material capable of being digested by the fungus. It is also known from US Patent 8,001,719 to enclose and grow a fungal primordium in a meld to obtain a mass of fungal tissue in the form of low density chitinous material.

It is an object of this invention to provide an improved method for the production of dehydrated mycelium elements.

It is another object of this invention to produce dehydrated mycelium pellets that can be used as is or can be used to make formed elements.

Briefly, the invention provides a method for producing dehydrated mycelium which can be rehydrated and rapidly re-formed into many different shapes, such as bricks, blocks, pellets and the like elements wherein the adhesion of the elements is achieved through reanimation of a fungal organism which grows the elements together.

In one embodiment, the invention provides a method of making dehydrated mycelium elements as set out in claim 1.

Described herein is a method of making dehydrated mycelium elements comprising the steps of creating a living hydrated mycelium composite containing at least one of a combination of mycelium and fibers, mycelium and particles, and mycelium, particles and fibers; adding a nutrient material to the mycelium composite in an amount to promote mycelia tissue growth; thereafter dehydrating the mycelium composite to a moisture content of less than 50% by weight to deactivate the further growth of mycelia tissue; and thereafter storing the dehydrated mycelium composite at a temperature in the range of from -50°F to +200°F.

The dehydrated mycelium composite may be processed into a plurality of discrete particles or coated fibers for storage.

The stored dehydrated mycelium composite may then be taken out of storage and further processed by adding moisture to the plurality of discrete particles in an amount sufficient to re-hydrate the particles and to re-activate mycelium on the exterior of the particles for growth into adjacent discrete particles.

Thereafter, the re-hydrated particles may be molded into at least one pellet or molded into at least one aggregated mass with mycelium bonding between and around the particles or re-incubated for a time sufficient for mycelium to bond particles together into a coherent mass.

Thereafter, the coherent mass may be dehydrated to a moisture content of from 0 to 30%, at a temperature greater than 150° F and for a time sufficient to permanently deactivate the mycelium.

In accordance with the method, dehydrated blocks and bricks can be formed which can be milled, cut, or otherwise transformed into new shapes. These shapes when re-hydrated will grow fresh exterior skins, and, when placed in contact, will self adhere to each other.

For example, the re-hydrated particles may first be fabricated into one or more panels, each of which is thereafter separated into a plurality of blocks (or cubes).

Moisture is then added to the blocks in an amount sufficient to re-hydrate blocks and to re-activate mycelium on the exterior of blocks for growth into adjacent blocks to bond the blocks together to form a fabricated section. A plurality of such fabricated sections may then be placed in direct contact with each other with mycelium on exterior surfaces of the sections bonding the sections together.

These and other objects and advantages of the invention will become more apparent from the following detailed description taken in conjunction with the accompanying drawings wherein:
Fig. 1 illustrates initial steps in a method of making mycelium pellets in accordance with the invention;
Fig. 2 illustrates further steps in the method of Fig. 1;
Fig. 3 illustrates a perspective view of a plank formed in accordance with the method described herein;
Fig. 4 illustrates an exploded view of a plurality of planks for forming a useful product in accordance with the method described herein;
Fig. 5 illustrates an exploded view of additional planks being added to the structure of Fig. 4;
Fig. 6 illustrates a perspective view of an assemblage of planks for forming a useful product prior to hydrating the assemblage; and
Fig. 7 illustrates a perspective view of the finished product made from the assemblage of Fig. 6.

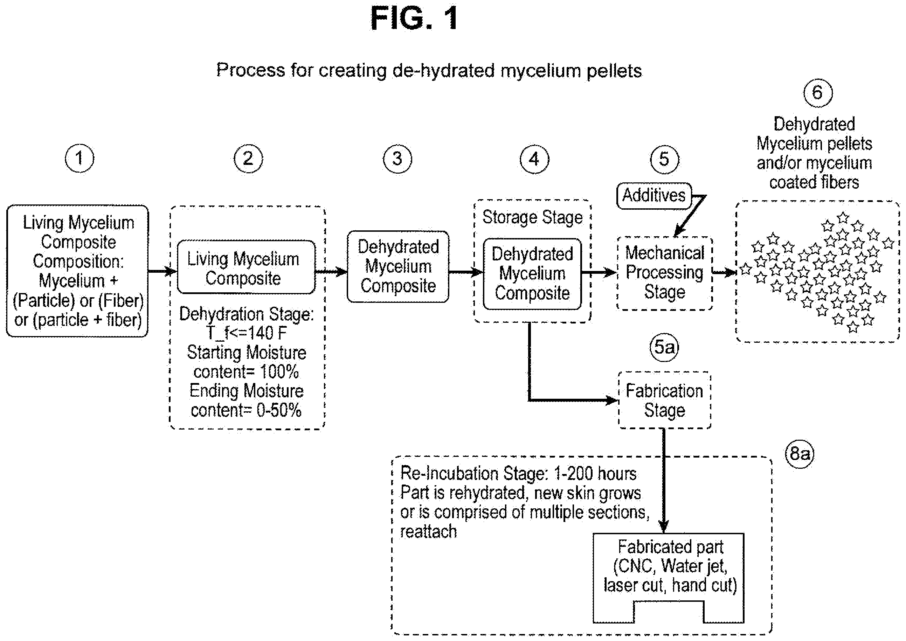

Referring to Fig. 1, in accordance with a first step 1, a living mycelium composite is created. This can be a combination of mycelium and fibers, mycelium and particles, or both mycelium, particles, and fibers. Generally, a nutrient material is included with mycelium and fibers to promote mycelia tissue growth, such as described in Serial No. 12/001,556, filed December 12, 2007.

In accordance with step 2, the living composite from step 1 is dehydrated at a temperature less than 140° F from a starting moisture content of 100% to an ending moisture content of less than 50%.In accordance with step 3, the dehydrated mycelium composite is now in-active, and will no longer grow mycelia fibers, and will be incapable of producing mushrooms, primordia, or other tissue.

In accordance with step 4, the dehydrated mycelium composite is placed in storage stage and stored indefinitely, at temperatures ranging from -50° F to 200° F. Lower moisture contents allow higher temperature storage ranges.

In accordance with step 5, the dehydrated mycelium composite from step 4 is mechanically processed into a plurality of particles or fibers in a mechanical processing stage. Additional additives, such as nutrients, grown enhancing compounds, binding agents, addition particles, additional fibers, or other materials may be added at this stage.

In accordance with step 6, the resulting particles and/or fibers geometry and size can be tuned to result in different densities and self adhesion characteristics. In the case of fibers, the fibers appear as being coated with mycelium.

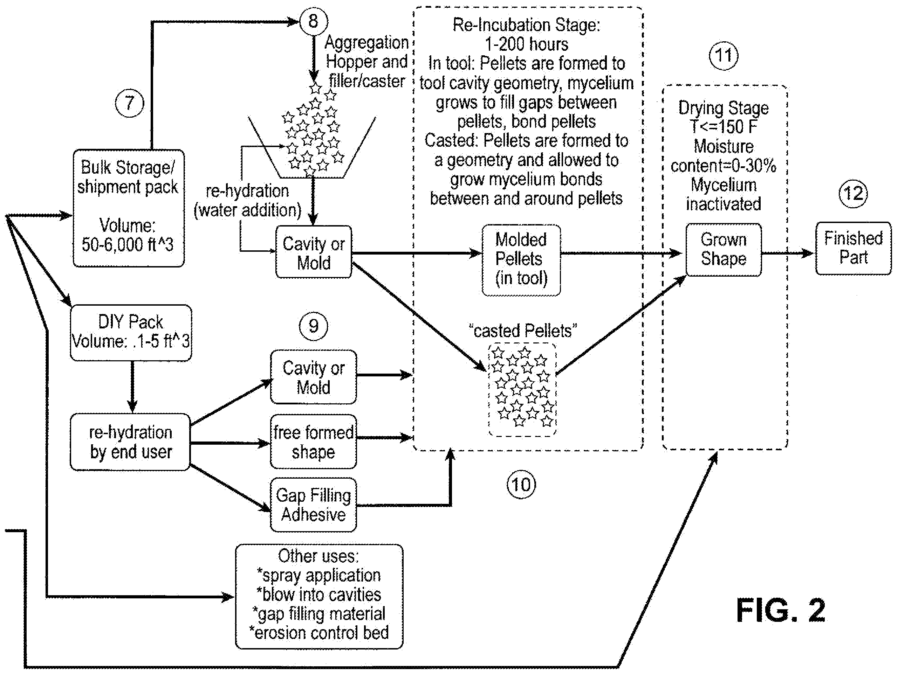

Referring to Fig. 2, in accordance with step 7, the dehydrated mycelium composite particles and/or fibers from step 5 are stored in bulk, for example, in volumes of from 50 to 6000 cubic feet in step 7 or packed into a variety of containers or transport vessels for distribution including bulk shipments, super-sacks, tractor trailers, or small DIY packages of from 1 to 5 cubic feet (e.g. in 1 to10 gallon containers).

Also, the dehydrated mycelium composite particles and/or fibers from step 5 may be used in other applications, such as for spray application, or by being blown into cavities and/or as gap filling material and in erosion control beds.

After storage and/or shipment, e.g. to a point of use, the dehydrated mycelium composite particles and/or fibers from step 7 are re-hydrated in accordance with step 8 to allow growth through the addition of moisture. Moisture can be added such that particles return to a 100% moisture, or just enough moisture can be added (often less than 10% of the starting volume) such that the mycelium on the exterior of the particles re-activities and is able to grow into adjacent particles.

In accordance with step 8, the dehydrated mycelium composite particles and/or fibers are delivered into an aggregation hopper and filler caster with water being added to the hopper, for example, by spraying. The re-hydrated mycelium composite particles and/or fibers are then delivered into a cavity or mold to be molded into elements, such as pellets.

In a similar manner, the dehydrated mycelium composite particles and/or fibers that are packaged in the small DIY packages can be rehydrated in a modified step 9 by the end user in any suitable manner and then placed in a cavity or mold to form an aggregated mass, or used as a free form shape or have an adhesive added to fill the gaps between the particles and/or fibers.

In accordance with step 10, the elements from step 9 are subject to a re-incubation stage for a period of time of from 1 to 200 hours in either a tool or are casted.

For example, the molded pellets from the cavity or mold of step 9 are formed to a tool cavity geometry and the mycelium allowed to grow to fill the gaps between the pellets. Alternatively, the pellets are formed to a geometry and allowed to grow mycelium bonds between the pellets.

In a similar manner, the pellets or elements formed from the dehydrated mycelium composite particles and/or fibers that are packaged in the small DIY packages are allowed to incubate and grow together in the re-incubation stage or may be casted into a free supporting form. An additive may be applied to promote initial adhesion, or may be added at stage 5.

In accordance with step 11, the grown shape from the re-incubation stage is once again dehydrated in a drying stage to a moisture content of from 0 to 30%, at a temperature greater than 150° F, such that the mycelium is permanently inactivated.

Thereafter, the resulting dehydrated shape is delivered in accordance with step 12 as a finished part suitable for use.

Alternately, in accordance with step 5a, the dehydrated mycelium composite from step 4 may be fabricated in a fabrication stage into panels and the dehydrated panels may be cut, for example, by CNC, water jet, laser cut, hand cut and the like, or otherwise transformed into a fabricated bulk shape, such as into blocks or bricks.

Thereafter, in accordance with step 8a, the mycelium composite fabricated blocks are subjected to a re-incubation stage wherein the blocks are rehydrated to grow fresh mycelium around joints that have been cut. As above described, re-incubation takes place over a time of from 1 to 200 hours whereby a part is rehydrated, new skin grows or, if comprised of multiple sections, reattach.

In addition, multiple fabricated sections may be placed in direct contact to promote growth adhesion between sections.

The entire block may be hydrated, or the exterior surfaces may be hydrated, both will promote exterior mycelia growth. The hydrated blocks are then dried as in step 11 and delivered as in step 12 as finished products.

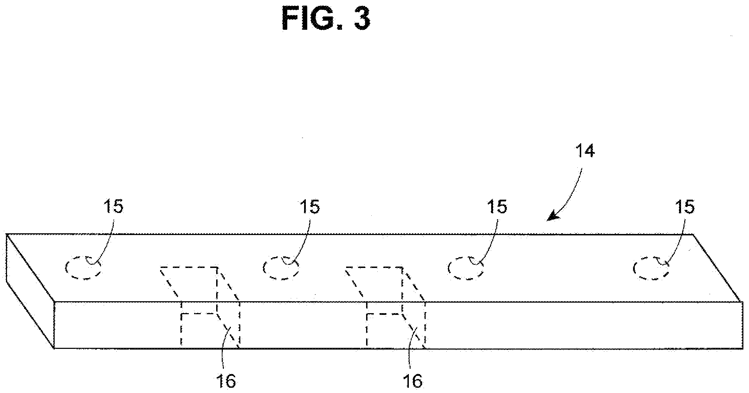

Referring to Fig. 3, a finished part 14 produced in accordance with the above-described method may be in the form of a dehydrated block 14 (hereinafter "mycoblock"). Such a mycoblock 14 may be drilled to form bores 15 extending therethrough at predetermined places and otherwise cut or machined to form recesses 16 at predetermined places.

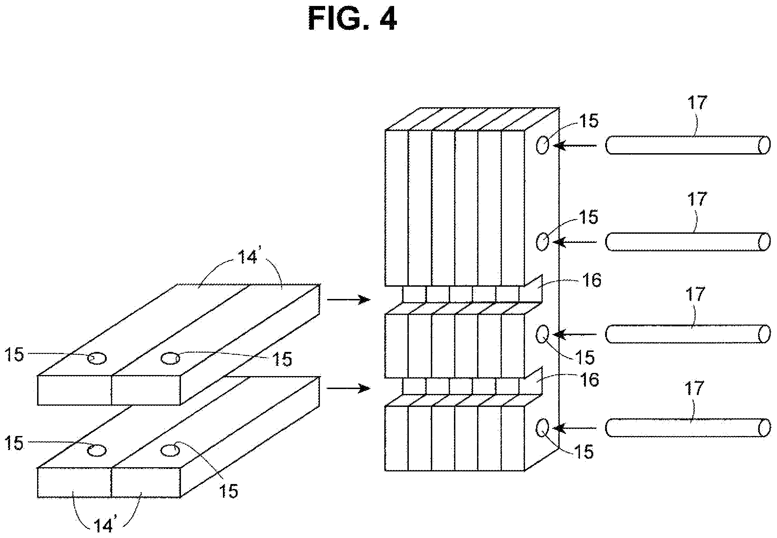

Referring to Fig. 4, in order to form a useful product, a plurality of mycoblocks 14, e.g. six, are stacked with the bores 15 in alignment and dowels 17 are passed into the aligned bores 15 to hold the mycoblocks 14 together. In addition, additional mycoboards 14' of a shorter length are placed together in pairs and inserted into the recesses 16 of the stacked mycoboards 14. As illustrated, each of the shorter mycoboards 14" has a bore 15 at an end opposite the end that is fitted into the stacked mycoboards 14.

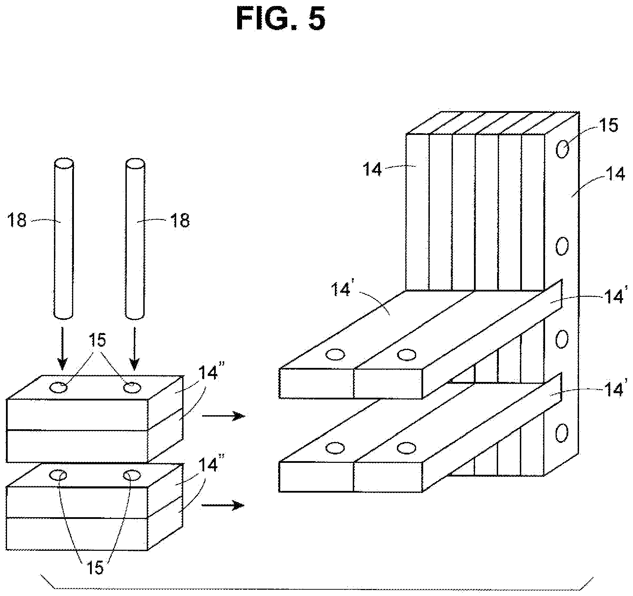

Referring to Fig. 5, in a further step, additional mycoboards 14" of even shorter length than the mycoboards 14', each with a pair of bores 15 are placed together in pairs and inserted between the free ends of the mycoboards 14' extending from the stacked mycoboards 14. In addition, dowels 18 are inserted in the bores 15 of the mycoboards 14" to hold them to the free ends of the mycoboards 14' and to complete an assemblage.

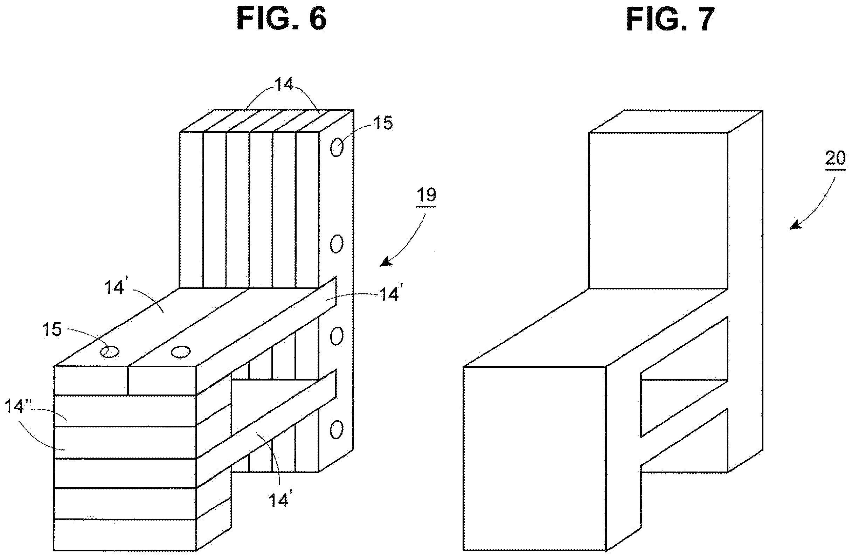

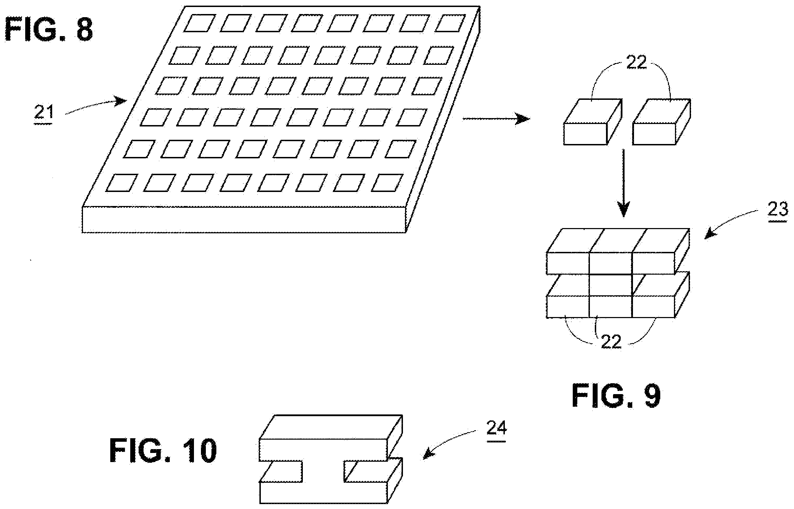

Referring to Fig. 6, the assemblage 19 of mycoboards 14, 14',14" is then subjected to rehydration wherein water is added to the assemblage to reanimate the fungus and the mycoboards bound into a cohesive unitary structure.

Referring to Fig. 7, the rehydrated assemblage 19 is allowed to incubate for a time, such as from 3 to 5 days, sufficient to form a self-supporting useful product 20 and is then dried, for example in sunlight, to inactivate the fungus.

The method described above may be summarized as making grown mycological composites, desiccating the materials, processing to create a uniform particle, and then reanimating the materials to create a new uniform solid.

The following presents a more detailed description of one embodiment of the method.

### Production of the Tissue Culture

A solid grain carrier was used as the predominate substrate for inoculation. The grain spawn was grown and prepared in batches of five gallons of dedicated grain spawn on a weekly basis. Each gallon of grain spawn was comprised of 800g of yellow millet, 600 mL of de-ionized water, and 10g of gypsum. Bags of the grain spawn were sterilized in an autoclave at 240°F and 15 psi for one hour and then allowed to cool to room temperature in a HEPA filtered laminar flow hood.

The bags of grain were then inoculated with the mycelium culture (tenth of a 100 mm Petri dish culture per bag) and incubated in ambient lab conditions and full colonization was achieved in five to seven days.

### Determination of Optimal Method for Particle Production

The first iteration of this task used 20L of each substrate, fiberized cotton gin waste and oat hulls, that were sterilized using an autoclave and then inoculated with grain spawn.

The bags were inoculated in a HEPA filtered laminar flow hood at a 20% [m:m] rate, and then incubated until colonization was complete.

Once colonization was achieved the materials were desiccated in a laminar flow hood at ambient conditions over the course of four days. The materials were separated bagged and labeled and sent to the USDA Agricultural Research Service in Lubbock, TX, for machine processing. The process equipment included a hammer mill (0.25" and 1" screen sizes), a rotary shear cutter (dual axis with helical cutters), screen-classified cutter (single axis with knife edges at the edge of the hopper), and a Titan shear (dual axis with cutters mounted on the shafts, rotating inward).

The results from a first iteration found that the dehydrated blocks processed in the hammer mill offered the best mycelium reanimation with the least residual contamination when incubated on an agarose media (MEA). These blocks, however, were not uniform in moisture content due to the amorphous form factor and as such this portion of the study was repeated using the same methods and equipment.

### Particle Moisture Content and Viability

In determining moisture content, 15 L of each substrate type was sterilized in an autoclave and then inoculated with *Ganoderma resinaceum* grain spawn. The inoculated substrate was evenly divided by mass into 18 tools per substrate (12" x 5" x 1"). All replicates, 36 total, were incubated until colonization was complete after seven days.

Each set of 18 was divided into three sets of six, representing three drying temperatures (i.e. 24°C, 53°C, and 82°C). The materials desiccated at room temperature were placed in a HEPA filtered laminar flow hood (forced convection), while the other two sets were dried in a programmed Despatch Convection Dryer that is positively pressurized with HEPA filtered air.

Each set of six was then measured for moisture content on an hourly basis using a conductivity meter, until two replicates each achieved 10%, 30%, and 50% moisture content by weight.

Once the drying cycles were complete each of the 18 sets were evenly divided by mass into six separate storage bags, representing six months of the reanimation study. The dehydrated particles were then placed on PDA and TSA, which are selective for molds and bacteria selectively. For the fiberized cotton burr particles the least contamination was observed on the sets dried at 82°C, including no bacteria contamination, and the best recovery was found with the materials desiccated at ambient temperatures. The oat hull sets had less contamination and better growth over all when compared to the fiberized cotton burr. The best desiccation level initially appears to be 30% moisture with a drying temperature around 53°C.

The particle viability study sought to determine the optimal storage time for the desiccated particles based on dehydration temperature, stored moisture content, and substrate. The two primary sets were divided into sterilized substrate and plant essential oil disinfected substrate. The two sets were analyzed over the course of six months measuring conducting a binary growth analysis, contamination observations, and radial growth over after five days of incubation from the inoculation point. All six months of the sterilized sets have been completed and the plant essential oil sets have been completed for the first four months.

The optimal moisture content and drying temperature for storage is 10% at 53°C for all sets with the exception of the fiberized burr substrate disinfected with a plant essential oil emulsion. The 50% moisture sets predominately harbors microbial contaminates, such as bacteria and mold, and as such should not be used for long-term storage. The materials disinfected with the plant essential oils have a better range of dehydration temperatures and moisture contents for storage, since more of the sets exhibited reanimation.

The invention thus provides a relatively simple and economic method of making dehydrated mycelium elements that can be used as is or in the subsequent fabrication of various shaped parts.

The dehydrated block of the invention which may be re-hydrated is distinguished from a dehydrated block of the invention that is not capable of being re-hydrated in that the block which may be re-hydrated is characterized in having a moisture content of less than 30% and in having the mycelium therein in a de-activated state capable of being re-activated for growth upon the addition of moisture whereas a dehydrated block of the invention that is not capable of being re-hydrated is characterized in having the mycelium in a permanently de-activated state that is not capable of being re-activated for growth.

## Claims

1. A method of making dehydrated mycelium elements comprising the steps of:
(1) creating a living hydrated mycelium composite containing at least one of a combination of mycelium and fibers, mycelium and particles, and mycelium, particles and fibers;
adding a nutrient material to said mycelium composite in an amount to promote mycelia tissue growth;
thereafter dehydrating the mycelium composite to a moisture content of less than 50 % by weight to deactivate the further growth of mycelia tissue to form a dehydrated mycelium element;
thereafter storing the dehydrated mycelium element at a temperature in the range of from -50 °F to +200 ºF (-45.56 to 93.33 ºC); and
adding moisture to said mycelium element in an amount sufficient to rehydrate said mycelium element and to re-activate mycelium on the exterior of said mycelium for growth into an adjacent mycelium element to bond said elements together to form a fabricated section; OR
(2) creating a living hydrated mycelium composite containing at least one of a combination of mycelium and fibers, mycelium and particles, and mycelium, particles and fibers;
adding a nutrient material to said mycelium composite in an amount to promote mycelia tissue growth;
thereafter dehydrating the mycelium composite to a moisture content of less than 50% by weight to deactivate the further growth of mycelia tissue;
processing the dehydrated mycelium composite into a plurality of discrete particles; and
adding moisture to said plurality of discrete particles in an amount sufficient to re-hydrate said discrete particles and to re-activate mycelium on the exterior of said discrete particles for growth Into adjacent discrete particles.

2. A method as set forth in claim 1, part (1), wherein a plurality of said fabricated sections are placed in direct contact with each other with mycelium on exterior surfaces of said sections bonding said sections together.

3. A method as set forth in claim 1, part (2) of making dehydrated mycelium elements comprising the steps of
creating a living hydrated mycelium composite containing at least one of a combination of mycelium and fibers, mycelium and particles, and mycelium, particles and fibers;
adding a nutrient material to said mycelium composite in an amount to promote mycelia tissue growth;
thereafter dehydrating the mycelium composite to a moisture content of less than 50% by weight to deactivate the further growth of mycelia tissue;
processing the dehydrated mycelium composite into a plurality of discrete particles; and
adding moisture to said plurality of discrete particles in an amount sufficient to re-hydrate said discrete particles and to re-activate mycelium on the exterior of said discrete particles for growth Into adjacent discrete particles.

4. A method as set forth in claim 3 further comprising the step of adding an additive to said plurality of discrete particles selected from the group consisting of nutrients, grown enhancing compounds, binding agents, additional particles and additional fibers particles.

5. A method as set forth in claim 3 wherein said moisture is added to said plurality of discrete particles in the form of a spray of water.

6. A method as set forth in claim 3 further comprising the step of molding said rehydrated discrete particles into at least one pellet.

7. A method as set forth in claim 3 further comprising the step of molding said rehydrated discrete particles into at least one aggregated mass of particles with mycelium bonding between and around said particles.

8. A method as set forth in claim 3 wherein said re-hydrated discrete particles are re-incubated for a time sufficient for mycelium to bond said particles together into a coherent mass.

9. A method as set forth in claim 8 wherein said time is from 1 to 200 hours.

10. A method as set forth in claim 8 further comprising the step of thereafter dehydrating said mass to a moisture content of from 0 to 30 %, at a temperature greater than 150 °F (65.56 ºC) and or a time sufficient to permanently de-activate the mycelium.

11. A method as set forth in claim 3 further comprising the steps of fabricating said plurality of discrete particles into at least one panel and thereafter separating at least one of said panels into a plurality of blocks.

12. A method as set forth in claim 11 further comprising the step of adding moisture to said plurality of blocks in an amount sufficient to re-hydrate said blocks and to re-activate mycelium on the exterior of said blocks for growth Into adjacent blocks of said plurality of blocks to bond said blocks together to form a fabricated section.

13. A method as set forth in claim 12 wherein a plurality of said fabricated sections are placed in direct contact with each other with mycelium on exterior surfaces of said sections bonding said sections together.

## Patentansprüche

1. Verfahren zum Herstellen dehydratisierter Myzelelemente, umfassend die Schritte des:
(1) Erzeugens eines lebenden hydratisierten Myzelverbundstoffs, der mindestens eine von einer Kombination von Myzel und Fasern, Myzel und Teilchen und Myzel, Teilchen und Fasern enthält;
Zusetzens eines Nährstoffmaterials zu dem Myzelverbundstoff in einer Menge, um das Myzelgewebewachstum zu unterstützen;
daraufhin Dehydratisierens des Myzelverbundstoffs auf einen Feuchtigkeitsgehalt von weniger als 50 Gew.-%, um das weitere Wachstum von Myzelgewebe zu deaktivieren, um ein dehydratisiertes Myzelelement zu bilden;
daraufhin Speicherns des dehydratisierten Myzelelements bei einer Temperatur im Bereich von -50 °F bis +200 °F (-45,56 bis 93,33 °C);
Zusetzens von Feuchtigkeit zu dem Myzelelement in einer Menge, die ausreicht, um das Myzelelement zu rehydratisieren und das Myzel auf der Außenseite des Myzels für das Wachstum in ein anliegendes Myzelelement zu reaktivieren, um die Elemente zusammenzubinden, um einen hergestellten Abschnitt zu bilden; oder
(2) Erzeugens eines lebenden hydratisierten Myzelverbundstoffs, der mindestens eine von einer Kombination von Myzel und Fasern, Myzel und Teilchen und Myzel, Teilchen und Fasern enthält;
Zusetzens eines Nährstoffmaterials zu dem Myzelverbundstoff in einer Menge, um das Myzelgewebewachstum zu unterstützen;
daraufhin Dehydratisierens des Myzelverbundstoffs auf einen Feuchtigkeitsgehalt von weniger als 50 Gew.-%, um das weitere Wachstum von Myzelgewebe zu deaktivieren;
Verarbeitens des dehydratisierten Myzelverbundstoffs in eine Mehrzahl einzelner Teilchen; und
Zusetzens von Feuchtigkeit zu der Mehrzahl einzelner Teilchen in einer Menge, die ausreicht, um die einzelnen Teilchen zu rehydratisieren und das Myzel auf der Außenseite der einzelnen Teilchen für das Wachstum in anliegende einzelne Teilchen zu reaktivieren.

2. Verfahren wie in Anspruch 1, Teil (1) aufgeführt, wobei eine Mehrzahl der hergestellten Abschnitte in direkten Kontakt miteinander mit Myzel auf Außenflächen der Abschnitte, die die Abschnitte zusammenbinden, positioniert wird.

3. Verfahren wie in Anspruch 1, Teil (2) aufgeführt, des Herstellens von dehydratisierten Myzelelementen, umfassend die Schritte des
Erzeugens eines lebenden hydratisierten Myzelverbundstoffs, der mindestens eine von einer Kombination von Myzel und Fasern, Myzel und Teilchen und Myzel, Teilchen und Fasern enthält;
Zusetzens eines Nährstoffmaterials zu dem Myzelverbundstoff in einer Menge, um das Myzelgewebewachstum zu unterstützen;
daraufhin Dehydratisierens des Myzelverbundstoffs auf einen Feuchtigkeitsgehalt von weniger als 50 Gew.-%, um das weitere Wachstum von Myzelgewebe zu deaktivieren;
Verarbeitens des dehydratisierten Myzelverbundstoffs in eine Mehrzahl einzelner Teilchen; und
Zusetzens von Feuchtigkeit zu der Mehrzahl einzelner Teilchen in einer Menge, die ausreicht, um die einzelnen Teilchen zu rehydratisieren und das Myzel auf der Außenseite der einzelnen Teilchen für das Wachstum in anliegende einzelne Teilchen zu reaktivieren.

4. Verfahren wie in Anspruch 3 aufgeführt, ferner den Schritt umfassend des Zugebens eines Zusatzmittels zu der Mehrzahl einzelner Teilchen ausgewählt aus der Gruppe bestehend aus Nährstoffen, wachstumsfördernden Verbindungen, Bindungsmitteln, zusätzlichen Teilchen und zusätzlichen Faserteilchen.

5. Verfahren wie in Anspruch 3 aufgeführt, wobei die Feuchtigkeit zu der Mehrzahl einzelner Teilchen in Form eines Wassersprays zugegeben wird.

6. Verfahren wie in Anspruch 3 aufgeführt, umfassend ferner den Schritt des Formens der rehydratisierten einzelnen Teilchen in mindestens ein Kügelchen.

7. Verfahren wie in Anspruch 3 aufgeführt, ferner den Schritt des Formens der rehydratisierten einzelnen Teilchen zu mindestens einer aggregierten Masse von Teilchen mit Myzelbindung zwischen den und um die Teilchen umfassend.

8. Verfahren wie in Anspruch 3 aufgeführt, wobei die rehydratisierten einzelnen Teilchen für eine Zeitspanne erneut inkubiert werden, die ausreicht, damit das Myzel die Teilchen zu einer zusammenhängenden Masse zusammenbindet.

9. Verfahren wie in Anspruch 8 aufgeführt, wobei die Zeit 1 bis 200 Stunden beträgt.

10. Verfahren wie in Anspruch 8 aufgeführt, ferner den Schritt des darauffolgenden Dehydratisierens der Masse auf einen Feuchtigkeitsgehalt von 0 bis 30 % bei einer Temperatur von nicht mehr als 150 °F (65,56 °C) und für eine Zeit, die ausreicht, das Myzel auf Dauer zu deaktivieren, umfassend.

11. Verfahren wie in Anspruch 3 aufgeführt, ferner die Schritte des Verarbeitens der Mehrzahl einzelner Teilchen zu mindestens einer Platte und daraufhin das Trennen mindestens einer der Platten in eine Mehrzahl von Blöcken umfassend.

12. Verfahren wie in Anspruch 11 aufgeführt, ferner den Schritt des Zugebens von Feuchtigkeit zu der Mehrzahl von Blöcken in einer Menge umfassend, die ausreicht, um die Blöcke zu rehydratisierten und das Myzel auf der Außenseite der Blöcke zum Wachstum in aneinanderliegende Blöcke der Mehrzahl von Blöcken zu reaktivieren, um die Blöcke zusammenzubinden, um einen hergestellten Abschnitt zu bilden.

13. Verfahren wie in Anspruch 12 aufgeführt, wobei eine Mehrzahl der hergestellten Abschnitte in direktem Kontakt miteinander mit Myzel auf Außenflächen der Abschnitte, das die Abschnitte zusammenbindet, positioniert wird.

## Revendications

1. Un procédé de fabrication d'éléments de mycélium déshydratés comprenant les étapes suivantes :
(1) création d'un composite de mycélium hydraté vivant contenant au moins l'un parmi une combinaison de mycélium et de fibres, de mycélium et de particules et de mycélium, de particules et de fibres ;
ajout d'une matière nutritive audit composite de mycélium en une quantité pour favoriser la croissance du tissu mycéliaire ;
ensuite, déshydratation du composite de mycélium à une teneur en humidité inférieure à 50 % en poids pour désactiver la croissance ultérieure du tissu mycéliaire pour former un élément de mycélium déshydraté ;
ensuite, stockage de l'élément de mycélium déshydraté à une température dans la plage de -50 °F à +200 °F (-45,56 à 93,33 °C) ; et
ajout d'humidité audit élément de mycélium en une quantité suffisante pour réhydrater ledit élément de mycélium et pour réactiver le mycélium sur l'extérieur dudit mycélium, pour faciliter la croissance dans un élément de mycélium adjacent pour lier ensemble lesdits éléments pour former une section fabriquée ; ou
(2) création d'un composite de mycélium hydraté vivant contenant au moins l'un parmi une combinaison de mycélium et de fibres, de mycélium et de particules et de mycélium, de particules et de fibres ;
ajout d'une matière nutritive audit composite de mycélium en une quantité pour favoriser la croissance du tissu mycéliaire ;
ensuite, déshydratation du composite de mycélium à une teneur en humidité de moins de 50 % en poids pour désactiver la croissance ultérieure du tissu mycéliaire ;
traitement du composite de mycélium déshydraté en une pluralité de particules discrètes ; et
ajout d'humidité à ladite pluralité de particules discrètes en une quantité suffisante pour réhydrater lesdites particules discrètes et pour réactiver le mycélium sur l'extérieur des dites particules discrètes, destinée à la croissance en des particules discrètes adjacentes.

2. Un procédé comme indiqué dans la revendication 1, partie (1), dans lequel une pluralité des dites sections fabriquées sont placées en contact direct les uns avec les autres avec le mycélium sur les surfaces extérieures des dites sections liant ensemble lesdites sections.

3. Un procédé comme indiqué dans la revendication 1, partie (2), de fabrication d'éléments de mycélium déshydraté comprenant les étapes suivantes :
création d'un composite de mycélium hydraté vivant contenant au moins l'un parmi une combinaison de mycélium et de fibres, de mycélium et de particules et de mycélium, de particules et de fibres ;
ajout d'une matière nutritive audit composite de mycélium en une quantité pour favoriser la croissance du tissu mycéliaire ;
ensuite, déshydratation du composite de mycélium à une teneur en humidité de moins de 50 % en poids pour désactiver la croissance ultérieure du tissu mycéliaire ;
traitement du composite de mycélium déshydraté en une pluralité de particules discrètes ; et
ajout d'humidité à ladite pluralité des particules discrètes en une quantité suffisante pour réhydrater lesdites particules discrètes et réactiver le mycélium sur l'extérieur des dites particules discrètes, destinée à la croissance en des particules discrètes adjacentes.

4. Un procédé comme indiqué dans la revendication 3, comprenant l'étape d'ajout d'un additif à ladite pluralité de particules discrètes sélectionnées parmi le groupe composé d'éléments nutritifs, de composés d'amélioration de croissance, d'agents de liaison, de particules supplémentaires et de particules de fibres supplémentaires.

5. Un procédé comme indiqué dans la revendication 3, dans lequel l'humidité est ajoutée à ladite pluralité de particules discrètes sous la forme d'une pulvérisation d'eau.

6. Un procédé comme indiqué dans la revendication 3, comprenant en outre l'étape de moulage des dites particules discrètes réhydratées dans au moins une granule.

7. Un procédé comme indiqué dans la revendication 3, comprenant en outre l'étape de moulage des dites particules discrètes réhydratées dans au moins une masse agrégée de particules comportant une liaison de mycélium entre et autour des dites particules.

8. Un procédé comme indiqué dans la revendication 3, dans lequel lesdites particules discrètes réhydratées sont réincubées pendant une période suffisante pour que le mycélium se lie ensemble avec les particules, en une masse cohérente.

9. Un procédé comme indiqué dans la revendication 8, dans lequel le temps va de 1 à 200 heures.

10. Un procédé comme indiqué dans la revendication 8, comprenant en outre l'étape de déshydratation par la suite de ladite masse à une teneur en humidité de 0 à 30 %, à une température supérieure à 150 °F (65,56 °C) et/ou un temps suffisant pour désactiver définitivement le mycélium.

11. Un procédé comme indiqué dans la revendication 3, comprenant en outre les étapes de fabrication des dites particules discrètes dans au moins un panneau et ensuite, la séparation d'au moins un des dits panneaux en une pluralité de blocs.

12. Un procédé comme indiqué dans la revendication 11, comprenant en outre l'étape d'ajout d'humidité à ladite pluralité de blocs en une quantité suffisante pour réhydrater lesdits blocs et pour réactiver le mycélium sur l'extérieur des dits blocs, destinée à la croissance dans les blocs adjacents de ladite pluralité de blocs pour lier ensemble lesdits blocs pour former une section fabriquée.

13. Un procédé comme indiqué dans la revendication 12, dans lequel une pluralité des dites sections fabriquées sont placées en contact direct les uns avec les autres avec le mycélium sur les surfaces extérieures des dites sections liant ensemble lesdites sections.
